Europäisches Patentamt

European Patent Office

Office européen des brevets

(11) Publication number: **0 213 357**
**A2**

## (12) EUROPEAN PATENT APPLICATION

(21) Application number: 86109901.8

(22) Date of filing: 18.07.86

(51) Int. Cl.4: **A23K 1/165** , A23K 1/18 , C12P 21/02 , C12N 15/00

The applicant has filed a statement in accordance with Rule 28 (4) EPC (issue of a sample only to an expert).

(30) Priority: 22.07.85 JP 161428/85
02.10.85 JP 219921/85
26.12.85 JP 294883/85

(43) Date of publication of application:
11.03.87 Bulletin 87/11

(84) Designated Contracting States:
DE FR GB IT

(71) Applicant: KYOWA HAKKO KOGYO KABUSHIKI KAISHA
6-1, Ohtemachi Itchome
Chiyoda-ku Tokyo(JP)

(72) Inventor: Itoh, Seiga
218-14, Aza Hachimannishi Aihara
Kanagawa-ken(JP)
Inventor: Abe, Toshio
7-11-11, Makuharihongo
Chiba-shi Chiba-ken(JP)
Inventor: Ueda, Tetsuya
4373-4, Ino
Toride-shi Ibaraki-ken(JP)
Inventor: Kawauchi, Hiroshi 124, Aza
Sugishita Okirai
Sanriku-cho Kesen-gun
Iwate-ken(JP)

(74) Representative: Casalonga, Alain et al
BUREAU D.A. CASALONGA OFFICE JOSSE &
PETIT Morassistrasse 8
D-8000 München 5(DE)

(54) **Method for culturing fish and shellfish.**

(57) Method for culturing fishes or shellfishes using growth hormones is disclosed.

Further, a feed for culturing fishes containing a growth hormone carried homogeneously on a water insoluble carrier and a feed for culturing shellfishes containing a growth hormone are dislosed.

## Method for culturing fish and shellfish

Background of the Invention

The present invention relates to a method for culturing fishes and shellfishes by administering a growth hormone of a fish or a bird to the fishes and shellfishes.

It has been revealed by recent studies that growth hormones of fishes such as salmon and eel are polypeptides. Methods for effectively using such growth hormone polypeptides for growing fishes have not been developed yet.

The present inventors have studied about effective use of the growth hormones. As the result, the present inventors have found that fishes can be grown rapidly by dipping the fishes in a solution or a suspension containing a growth hormone polypeptide of a fish or a bird.

It has been known that a growth hormone of a fish is present in the pituitary gland of a fish. It is difficult to extract a growth hormone from the pituitary gland and to separate it from growth hormone analogues such as prolactin because of similarity of molecular weight and properties.

The present inventors isolated fish growth hormones from the pituitary gland of salmons and eels and established a method for producing the growth hormones by cultivating a microorganism of _Escherichia coli_ constructed by recombinant DNA technology (Japanese Published Unexamined Patent Application No. 214798/85, Japanese Patent Application Nos. 151847/85, 50096/85 and 161429/85).

Even if fishes are cultured under the same conditions in the same water tank, the growth ratios of the fishes are different. For example, in the case of a typical freshwater fish such as rainbow trout, even when fertilized eggs obtained by fertilizing eggs from one female fish with sperms from one male fish, the weight of the biggest fish after 6 months is about three times that of the smallest one. In the case of a marine fish, for example, red sea bream, when advanced fry obtained on the same day by fertilization of eggs from one female fish with sperms from one male fish and hatching are cultured for 3 months, the weight of the biggest fish is 21.2g and the smallest is 3.5g, i.e. the weight of the biggest fish is about 6 times that of the smallest one.

Thus, even when fishes derived from one couple of fishes are cultured under the same circumstances, the weight of the fishes are extremely varied and their abilities of swimming are also varied. Such differences among individuals result in the differences in food intake and competition. As the result, the difference in growth among the individuals increases, and repeated divisional cultures are required depending on the difference of the body weights. Such culture process requires much labor and excessive facilities and the fish bodies are sometimes injured during division procedure, which is liable to cause disease.

It is assumed that the use of a growth hormone of a fish can overcome the problems mentioned above. However, the growth hormone is slightly soluble in water and uptake thereof by fishes is restricted. Therefore, a method for introducing effectively the growth hormone into fish bodies has been demanded.

The present inventors have studied about a method for introducing effectively the growth hormone into fish bodies and found that the hormone can be taken by dipping fishes in a solution or a suspension containing the hormone and that the hormone carried homogenously on water-insoluble proteins is effectively taken in fish bodies orally.

Recently, culture of conchs such as abalones and top shells and bivalues such as hard clam, little clam and ark shell by artificial liberation has been developed and a method for accelerating the growth of shellfishes is demanded.

The present inventors have found that it is possible to accelerate effectively the growth of shellfishes by dipping them in a solution containing a growth hormone of a fish or by using a feed containing a growth hormone of a fish.

Summary of the Invention

The present invention provides a method for culturing fishes and shellfishes by administering a growth hormone of a fish or a bird to the fishes and shellfishes.

Further, the present invention provides a feed for fishes which contains a fish growth hormone carried homogeneously on a water-insoluble carrier and a feed for shellfishes which contains a fish growth hormone.

Detailed Description of the Invention

According to the present invention, a growth hormone is taken into fishes especially juvenile fishes, larval fishes and adolescent fishes by dipping them in a solution or a suspension of the growth hormone polypeptide, and rapid growth of the fishes is expected.

As the growth hormone polypeptide used in the present invention, any metabolic hormone which regulates metabolism is applicable. For example, metabolic hormones such as various growth hormones, prolactin, etc. obtained by the extraction from the pituitary gland of natural fishes, various growth hormones obtained by cultivating a transformant established by incorporating a gene coding for the growth hormone, etc. into a host microorganism using recombinant DNA technonolgy, and a derivative wherein a part of amino acid sequence of the growth hormone is deleted or replaced with other amino acids are all applicable in the present invention.

As the growth hormone used in the present invention, those originated from fishes are preferable and those originated from birds such as fowls are also applicable. Preferable examples are growth hormones and prolactins obtained by extraction from the pituitary gland of salmons, eels, carp, sea breams by the method of Farmer, et al. - [Gen. Comp. Endocrin., 30, 91 (1976)] and growth hormone polypeptides and prolactin polypeptides of salmons and eels produced by recombinant DNA technology. Salmon growth hormone polypeptides produced by recombinant DNA technology is referred to as r-SGH hereinafter. Generally, these growth hormone polypeptides have a molecular weight of about 20,000 to 30,000 and about 180 to 190 amino acid residues.

Examples of the growth hormone and processes for production thereof are described in Examples and Reference Examples below.

Uptake of a growth hormone into a fish is effected by dipping a fish in a solution or a suspension containing the growth hormone for a definite time or by culturing a fish in a solution or a suspension containing the growth hormone. Macromolecular hormones such as r-SGH are generally slightly soluble in water. In such case, in order to increase the solubility, a surfactant such as a glycerol fatty acid ester, a propyleneglycol fatty acid ester, a sorbitan fatty acid ester or a sucrose fatty acid ester is added in an amount of 5 -6%. In order to increase the uptake of the growth hormone into fishes, hypertonic water is applicable to culturing so far as it is harmless to fishes. Hypertonic water for culturing is generally obtained by elevating osmotic pressure of the water to about 1.0 to 1.5 times that of body fluid by adding about 2% or less sodium chloride or a sugar such as mannitol or sorbitol.

The concentration of the growth hormone in the solution or the suspension used for dipping fishes, the dipping temperature, the dipping time, etc. depend on species, age, etc. of the fishes. Generally, by dipping a fish in a solution or a suspension containing 1 -50 mg/ℓ growth hormone at a temperature lower than the culturing temperature, preferably lower by 5°C for about 5 minutes to 24 hours, the growth hormone is introduced into the fish, especially into blood through the skin, gill, lateral line, etc. The dipping treatment is carried out 1 -7 times at intervals of 4 -7 days.

The present invention also provides a feed for fishes, wherein a growth hormone of a fish is carried on a water-insoluble carrier.

As the growth hormone, those mentioned above are applicable.

As the water-insoluble carrier, proteins such as casein, glutelin and zein are employed. These proteins can be dissolved in a solvent under certain conditions. For example, casein is water-soluble in the form of sodium salt, glutelin is readily soluble in a slightly acidic or alkaline solvent, and zein is soluble in an alcohol. Therefore, it is possible to obtain a growth hormone-carrying water-insoluble protein wherein the growth hormone is homogenously dispersed in the protein or the protein is coated with the growth hormone by solubilizing the protein under suitable conditions, adding the growth hormone to the protein solution to dissolve or disperse the growth hormone therein and making the protein waterinsoluble. Purified proteins or proteins contained in raw fish meat paste are applicable.

The growth hormone is employed in a range of 10 mg to 10g per one kilogram of the water-insoluble carrier.

In the culturing of fishes, a synthetic food comprising a feed and the growth hormone carried on a water-insoluble carrier is orally fed to the fishes. The growth hormone in the synthetic food is used in a range of 1 to 1,000 mg/kg. The amount to be fed is depending on species of the fish, and usually is 0.1 -100 μg/g body weight/day.

Oral feeding is carried out by feeding a mixted feed, by injecting the growth hormone using a stomach catheter, or by introducing it directly into the stomach using gelatin capsules, etc.

The present invention is applicable to rainbow trout, dogsalmon, sweet fish, eel, flounder, red sea bream, black sea bream, carp, goldfish, top minnow, etc.

Further, the present invention provides a compound accelerating the growth of shellfishes, which contains a fish growth hormone. The growth of shellfishes can be accelerated by contacting the compound with the shellfishes.

As the shellfishes to which the present invention is applicable, conchs such as abalone, top shell, tokobushi abalone and mudsnail and bivalues such as hard clam, little clam, ark shell and pearl oyster are mentioned.

As the growth hormone, those mentioned above are applicable.

Contact of the growth hormone with shellfishes is carried out by dipping the shellfishes in a solution or a suspension containing the growth hormone for a definite time or by culturing the shellfishes in a water containing the growth hormone.

Fresh water, seawater or a hypertonic seawater is used for making the solution or suspension containing the growth hormone and for culturing the shellfishes.

The concentration of the growth hormone, the dipping temperature and the dipping time depend on species, age, etc. of the shellfishes. Generally, the shellfishes are dipped in a solution or a suspension containing 0.5 -50 mg/ℓ growth hormone at a temperature from the culturing temperature to that lower than the culturing temperature by about 5°C for about 5 minutes to 24 hours. Dipping is carried out generally at intervals of 3 -7 days from the shellfish age of 30 days till growth stops.

The growth hormone in the water for culturing is kept at a concentration of 0.01 -1 mg/ℓ.

Certain specific embodiments of the invention are illustrated by the following representative examples.

Example 1

Escherichia coli (FERM BP-612) carrying a plasmid coding for Oncorhynchus keta (referred to as salmon hereinafter) growth hormone (referred to as SGH hereinafter) was inoculated in 100 mℓ of MCG medium (pH 7.2) consisting of 0.6% Na$_2$HPO$_4$, 0.3% KH$_2$PO$_4$, 0.5% NaCl, 0.1% NH$_4$Cl, 0.5% glucose, 0.5% casamino acid, 1 mM MgSO$_4$ and 4 μg/mℓ vitamin B$_1$ and culturing is carried out at 30°C for 10 hours. The culture broth was centrifuged at 8,000 rpm for 10 minutes to collect cells. The cells were washed twice with 30 mM Tris-HCl buffer (pH 7.5) containing 30 mM NaCl and suspended in 100 mℓ of a medium having the same composition as the MCG medium described above except that 0.5% casamino acid is replaced with 0.5% a mixture of 19 amino acids other than methionine. The suspension was cultured with shaking at 30°C for 20 minutes and 50 μCi of L-[$^{35}$S]-methionine [>800 Ci/mmol, product of New England Nuclear Co.] was added followed by culturing with shaking for 60 minutes. The culture broth was centrifuged at 8,000 rpm for 10 minutes to collect cells. The cells were washed twice with 30 mM Tris-HCl buffer (pH 7.5) containing 30 mM NaCl. The washed cells were suspended in 10 mℓ of the buffer described above and subjected to ultrasonic disruption using Sonifier cell disruptor 200 (Branson Sonic Power Company) under output control of 2 at 0°C for 10 minutes.

The disrupted cells were centrifuged at 15,000 rpm for 30 minutes to obtain cell residues. r-SGH labelled with 35g was extracted and purified from the cell residues by the method of Marston et al. - [F.A.O. Marston et al.: Biotechnology 2, 800 (1984)].

Ten rainbow trout (aged 7 months after hatching, about 10g) and 10 red sea bream (aged 4 months after hatching, about 10g) were dipped in water containing 2 mg/ℓ salmon growth hormone labelled with $^{35}$S ($^{35}$S-r-SGH) at an osmotic pressure of 0.5 -1.5 times that of body fluid at 15°C (rainbow trout) or 20°C (red sea bream) for 30 minutes.

Blood was recovered from the fishes and 100 μℓ of the blood was dropped on a glass filter. After drying, 1.5 mℓ of a toluene scintillator wherein 8g of 2,5-diphenyloxazol and 800 mg of dimethyl-phenyloxazoylphenyloxazoylphenyl were dissolved in 2ℓ of toluene was added and radio activity was determined with liquid scintillation counter (product of Packard, Model 3320). The results are illustrated in Table 1 wherein the data are indicated by the amount of r-SGH per g of fish body weight.

## Table 1

| Osmotic pressure | | x 0.5 | x 1.0 | x 1.5 |
|---|---|---|---|---|
| Rainbow trout | measured part | liver | whole body | whole body |
| | $\dfrac{\mu g}{g \text{ (body weight)}}$ | 0.1 | 0.5 | 1.2 |
| Red sea bream | measured part | liver | whole body | whole body |
| | $\dfrac{\mu g}{g \text{ (body weight)}}$ | 0.1 | 0.7 | 1.5 |

Example 2

Escherichia coli EUPA1 (FERM BP-825) carrying a recombinant plasmid pUPA1 containing a cDNA coding for an eel growth hormone (referred to as eGH hereinafter) was used to prepare eel growth hormone labelled with $^{35}$S (referred to as $^{35}$S-r-eGH) by the same method as described in Example 1. Uptake of r-eGH into fish bodies was determined using the $^{35}$S-r-eGH by the same method as described in Example 1 and the effect of increasing the weight of the fishes was examined.

Groups of rainbow trout (aged 7 months after hatching, about 11g), eels (whitebait, about 0.2g), and sweet fishes (aged 26 days, about 24 mg), each group consisting of 300 individuals, were dipped in water containing 1 to 10 mg/$l$ $^{35}$S-r-eGH at the same osmotic pressure as that of body fluid at a temperature indicated in Table 2 for 30 minutes.

Twelve fishes were taken from each group and the amount of $^{35}$S-r-eGH taken into the blood of two fishes among them was measured by the same method as described in Example 1.

The remaining fishes (10 per one group) were cultured in well water at 15°C and 20°C for 40 days. At intervals of 4 days after the start of culturing, the fishes were dipped four times in water containing unlabelled eel growth hormone which was prepared using Escherichia coli EUPA1 by the same method as in Example 1 without isotope (Reference Example 2). The body weights of the fishes were measured after 40 days. The results are illustrated in Table 2.

Table 2

| Fish | Water tempera-ture (°C) | Increase of body weight (%) | | | Uptake of r-eGH µg/g (body weight) | | |
|---|---|---|---|---|---|---|---|
| | | Concentration of the eel growth hormone (mg/ℓ) | | | | | |
| | | 10 | 1 | 0 | 10 | 1 | 0 |
| Rainbow trout | 20 | 260 | 130 | 65 | 10.8 | 2.0 | 0 |
| | 15 | 160 | 83 | 50 | 9.5 | 1.9 | 0 |
| Eel | 25 | 280 | 180 | 110 | 25.0 | 4.0 | 0 |
| | 20 | 220 | 150 | 90 | 22.8 | 4.0 | 0 |
| Sweet fish | 20 | 250 | 200 | 120 | 7.5 | 0.9 | 0 |
| | 15 | 200 | 150 | 100 | 3.8 | 0.9 | 0 |

Example 3

Groups of flounders (aged 45 days, about 0.1g), red sea bream (aged 30 days, about 14 mg), salmons (aged 3 -4 months after hatching, about 1.5g), black sea bream (aged 30 days, about 15 mg), goldfishes (aged 50 days after hatching, about 200 mg) and top minnows (aged 20 days after hatching, about 3 mg), each group consisting of 200 individuals, were dipped in water containing red sea bream growth hormone, which was obtained from the pituitary gland of red sea bream by the method of Farmer, et al., in a concentration indicated in Table 3 at an osmotic pressure which is 1.2 times that of body fluid at a temperature shown in Table 3 for 3 hours. The fishes were cultured in well water at a temperature shown in Table 3 for 40 days. At intervals of 4 days after the start of culturing, the fishes were dipped four times in water containing sea bream growth hormone by the same method as described above. The body weights of the fishes were measured after 40 days. The results are illustrated in Table 3.

## Table 3

| Fish | Water temperature (°C) | Increase of body weight (%) | |
|---|---|---|---|
| | | Concentration of the bream growth hormone (mg/ℓ) | |
| | | 20 | 0 |
| Flounder | 12 | 255 | 108 |
| | 17 | 265 | 111 |
| Red sea bream | 20 | 263 | 120 |
| | 25 | 270 | 223 |
| Salmon | 10 | 287 | 115 |
| | 15 | 290 | 118 |
| Black sea bream | 20 | 270 | 123 |
| | 25 | 281 | 128 |
| Goldfish | 20 | 254 | 112 |
| | 25 | 271 | 217 |
| Top minnow | 23 | 210 | 100 |
| | 28 | 225 | 107 |

Example 4

Groups of rainbow trout (aged 7 months after hatching, about 10g), black sea bream (aged 30 days, about 14 mg), eels (whitebait, about 0.2g) and sweet fishes (aged 30 days, about 27 mg), each group consisting of 200 individuals, were dipped in water containing r-SGH prepared by the method described in Reference Example 1 in a concentration indicated in Table 4 at an osmotic pressure which is 1.5 times that of body fluid at a temperature shown in Table 4 for one hour and 2 hours. These fishes were cultured in well water at a temperature indicated in Table 4 for 10 days. Four days after the start of culturing, the fishes were dipped in water containing salmon growth hormone by the same method as described above. The body weights of the fishes were measured 10 days after the start of culturing. The results are illustrated in Table 4.

### Table 4

| Fish | Water temperature (°C) | Increase of body weight (%) | | | | |
|---|---|---|---|---|---|---|
| | | Concentration of r-SGH (mg/ℓ) | | | | |
| | | 10 | | 1 | | 0 |
| | | 1h | 2h | 1h | 2h | |
| Rainbow trout | 20 | 20 | 25 | 17 | 20 | 15 |
| | 15 | 28 | 35 | 19 | 23 | 16 |
| Black sea bream | 25 | 25 | 33 | 20 | 23 | 17 |
| | 20 | 40 | 50 | 30 | 38 | 22 |
| Eel | 28 | 90 | 150 | 80 | 90 | 50 |
| | 23 | 110 | 200 | 95 | 120 | 55 |
| Sweet fish | 20 | 70 | 100 | 70 | 90 | 40 |
| | 15 | 82 | 120 | 90 | 100 | 45 |

### Example 5

Groups of carp (aged 10 months after hatching, about 15g), goldfishes (about 2g) and top minnows (about 0.2g), each group consisting of 50 individuals, were treated and cultured by the same method as in Example 3 except that carp growth hormone prepared from the pituitary gland of carp by the method of Farmer et al . and having a purity of 90% was used instead of red sea bream growth hormone. The results are illustrated in Table 5.

## Table 5

| Fish | Water temperature (°C) | Increase of body weight (%) | |
|---|---|---|---|
| | | Concentration of carp growth hormone (mg/ℓ) | |
| | | 20 | 0 |
| Carp | 20 | 275 | 116 |
| | 25 | 281 | 119 |
| Gold fish | 20 | 254 | 112 |
| | 25 | 271 | 217 |
| Top minnow | 23 | 210 | 100 |
| | 28 | 225 | 107 |

### Example 6

Groups of salmon (aged 4 months after hatching, about 1.5g), red sea bream (aged 35 days, about 15 mg) and sweet fishes (aged 33 days, about 30 mg), each group consisting of 300 individuals, were treated and cultured by the same method as described in Example 2 except that fowl growth hormone prepared by recombinant DNA technology using Escherichia coli [L.M. Souga et al. J. Exp. Zool., 232 , 465 -473 (1984)] was used. The results are illustrated in Table 6.

## Table 6

| Fish | Water temperature (°C) | Increase of body weight (%) | |
|---|---|---|---|
| | | Concentration of fowl growth hormone (mg/ℓ) | |
| | | 150 | 0 |
| Salmon | 12 | 230 | 106 |
| | 17 | 143 | 109 |
| Red sea bream | 20 | 246 | 118 |
| | 25 | 250 | 121 |
| Sweet fish | 17 | 231 | 117 |
| | 20 | 248 | 120 |

Example 7

50 juvenile eels (whitebait, about 0.2g) were dipped in water containing eel prolactin extracted and purified from the pituitary gland of eels by the method of Farmer et al. mentioned above in a concentration indicated in Table 7 at an osmotic pressure which is 1.5 times that of body fluid at 15°C for 30 minutes. Thereafter, the fishes were liberated into seawater and behavior of the fishes was observed. The results are illustrated in Table 7.

## Table 7

| Concentration of eel prolactin (mg/ℓ) | Time after liberation into seawater | |
| --- | --- | --- |
| | 30 minutes | 5 hours |
| 10 | Normal | Normal |
| 1 | Normal | Normal |
| 0.1 | Slightly active | Normal |
| ·0 | One turns laterally, inactive as a whole | One died, and the others were normal |

### Example 8

Juvenile sweet fishes (50 per one group) (aged 130 days, average body length 63 mm, average body weight 1.6g) were cultured for 30 days. During the culturing, the feeds prepared in Reference Example 3 and containing 1, 10, 100, and 1000 μg/g SGH, respectively were administered orally in a concentration of 0.05 g/g body weight/day by a conventional method. The body weights of the fishes after 30 days from the start of culturing were measured. The results are illustrated in Table 8.

## Table 8

| Feed (amount of SGH mg/kg) | Average body weight after 30 days (g) | Increase of body weight (%) |
| --- | --- | --- |
| 1 | $6.8 \pm 0.4$ | 325 |
| 10 | $8.5 \pm 0.3$ | 431 |
| 100 | $8.1 \pm 0.4$ | 406 |
| 1000 | $7.2 \pm 0.3$ | 350 |
| Control | $5.5 \pm 0.6$ | 244 |

### Example 9

Groups of rainbow trout (aged 70 days, average body weight 0.5g), flounders (aged 55 days, average body weight 0.2g), red sea bream (aged 46 days, average body weight 0.1 g) and eels - (whitebait, average body weight 0.2g), each group consisting of 50 individuals, were cultured for 40 days.

During the culturing, the feed prepared in Reference Example 4 and containing 200 μg/g SGH was administered orally 8 times (twice a week) in a concentration of 0.05 g/g body weight. In the case of eels, the feed was administered orally in a conventional manner as a paste wherein salmon growth hormone was dissolved and mixed just before feeding. The body weights after 40 days from the start of culturing were measured. The results are illustrated in Table 9.

## Table 9

| Fish | Water temperature (°C) | Increase of body weight (%) | |
|---|---|---|---|
| | | Test | Control |
| Rainbow trout | 15 | 87 ± 13 | 61 ± 19 |
| Flounder | 20 | 128 ± 11 | 110 ± 21 |
| Red sea bream | 20 | 140 ± 10 | 123 ± 23 |
| Eel | 25 | 145 ± 12 | 120 ± 30 |

Example 10

Red sea bream, flounders and eels were cultured according to the method of Example 9 using the feed prepared by the method of Reference Example 5 using the eel growth hormone obtained by the method of Reference Example 2. The amount of the eel growth hormone added was 100 mg per/kg of feed. In the case of eels, the feed was administered orally in a conventional manner as a paste wherein the eel growth hormone was dissolved and mixed just before feeding. The results are illustrated in Table 10.

## Table 10

| Fish | Increase of body weight (%) | |
|---|---|---|
| | Test | Control |
| Red sea bream | 550 | 420 |
| Flounder | 720 | 510 |
| Eel | 430 | 215 |

Example 11

15 mg of salmon growth hormone produced by the method of Reference Example 1 and 15 mg of salmon prolactin produced by the method of Reference Example 6 were respectively dissolved in 2 mℓ of sodium deoxycholate and added to 500 mℓ of seawater. As a control, 2 mℓ of sodium deoxycholate was added to 500 mℓ of seawater. Groups of 12 abalone larvae (average length 11.9 ± 0.2 mm, average width 8.5 ± 0.2 mm) were respectively dipped in solutions containing the growth hormone and prolactin and a control solution, at room temperature for 30 minutes at intervals of 3 days.

After dipping, the shellfishes were moved into seawater and cultured under natural light at a water temperature of 19 -20°C. During culturing, 1.5g of synthetic feed for abalone (product of Nippon Nosan Kogyo Co.) was fed once a day in the morning. After the dipping treatment was repeated eight times, the increases of the length and width of the abalone larvae were measured. The results are illustrated in Table 11.

## Table 11

| Sample | Concentra-tion (μg/mℓ) | Increase of the shell length (mm) (exponent) | Increase of the shell width (mm) (exponent) |
|---|---|---|---|
| Control | 0 | 1.2($\pm$ 0.2)   100 | 0.6($\pm$ 0.1)   100 |
| Prolactin | 30 | 3.1($\pm$ 0.2)   258 | 1.7($\pm$ 0.2)   283 |
| Growth hormone | 30 | 4.3($\pm$ 0.2)   358 | 2.3($\pm$ 0.2)   383 |

Example 12

Groups of 10 top shell larvae (average length 16.6 mm, average weight 1.26g) were respectively dipped in a solution containing 30 μg/mℓ salmon growth hormone, a solution containing 30 μg/mℓ salmon prolactin, and a control solution of sodium deoxycholate, which were prepared in the same manner as in Example 11, for 30 minutes at intervals of 4 days.

After the dipping treatment, the shellfishes were moved into a rearing tank containing 100ℓ of seawater and culturing was continued in running water. The average water temperature during the culturing was 18.2°C, and raw Undaria was fed every four days. The test period was 30 days, during which 8 dipping treatments were carried out. The length and weight of the tops shells were measured after the culturing. The results are illustrated in Table 12.

Table 12

| Sample | Concen-tration (µg/mℓ) | Length (mm) | | | Weight (g) | | |
|---|---|---|---|---|---|---|---|
| | | Start | 30 days | Increase* exponent | Start | 30 days | Increase* exponent |
| Control | 0 | 16.6 | 16.8 | 100 | 1.27 | 1.34 | 100 |
| Growth hormone | 30 | 16.5 | 17.9 | 700 | 1.23 | 1.55 | 457 |
| Prolactin | 30· | 16.6 | 17.1 | 250 | 1.28 | 1.45 | 243 |

\* Increase exponent

$$= \frac{\text{The value of test after 30 days} - \text{the value of test at the start}}{\text{The value of control after 30 days} - \text{the value of control at the start}} \times 100$$

Example 13

Groups of 20 hard clam larvae (average length 10.5 mm, average weight 0.75g) were respectively dipped four times in a solution containing 20 µg/mℓ salmon growth hormone, a solution containing 20 µg/mℓ salmon prolactin, and a control solution of sodium deoxycholate, which were prepared in the same manner as in Example 11, for 30 minutes at intervals of one week. After each dipping, the shellfishes were moved into a rearing tank containing 100ℓ of seawater and a sand layer with a thickness of 10 cm at the bottom, and cultured in running water. The average water temperature during the culturing was 19.0 ± 1.0°C, and diatom which is a phytoplankton was fed sufficiently. The length and weight of the hard clams after culturing for 30 days were measured. The results are illustrated in Table 13.

Table 13

| Sample | Concen-tration (μg/mℓ) | Length (mm) | | | Weight (g) | | |
|---|---|---|---|---|---|---|---|
| | | Start | 30 days | Increase* exponent | Start | 30 days | Increase* exponent |
| Control | - | 10.5 | 10.6 | 100 | 0.75 | 0.77 | 100 |
| Growth hormone | 20 | 10.5 | 10.9 | 400 | 0.75 | 0.81 | 300 |
| Prolactin | 20 | 10.5 | 10.7 | 200 | 0.75 | 0.78 | 150 |

\* The increase exponent was calculated by the formula shown in Example 12.

Example 14

Groups of 20 little clam larvae (average length 0.8 mm) were respectively dipped four times in a solution containing 20 μg/mℓ salmon growth hormone, a solution containing 30 μg/mℓ salmon growth hormone, and a control solution of sodium deoxycholate, which were prepared in the same manner as in Example 11, for 30 minutes at intervals of one week. After each dipping, the shellfishes were moved into a rearing tank containing 100ℓ of seawater and a sand layer with a thickness of 10 cm at the bottom, and cultured in running water. The average water temperature during the culturing was 19.0 ± 1.0°C and diatom which is a phytoplankton was fed sufficiently. The length and weight of the little clams after culturing for 30 days were measured. The results are illustrated in Table 14.

## Table 14

| Sample | Concentration (μg/mℓ) | Length (mm) | | |
|---|---|---|---|---|
| | | Start | 30 days | Increase* exponent |
| Control | - | 0.8 | 0.85 | 100 |
| Growth hormone | 20 | 0.8 | 0.88 | 130 |
| | 30 | 0.8 | 0.89 | 145 |

\* The increase exponent was calculated by the formula shown in Example 12.

Reference Example 1

Production of salmon growth hormone:

Escherichia coli W3110 No. 49 was transformed with recombinant plasmid pSGHIB2 isolated from Escherichia coli ESGHIB2 (FERM BP-612) by the method of Birnboim et al. [H.C. Birnboim et al.: Nucleic Acids Res. 7, 1513 (1979)]. The transformation was carried out by a conventional method [Proc. Natl. Acad. Sci., USA 69, 2110 (1972)]. The obtained ampicillin-resistant colony was inoculated in 8 mℓ of MCG medium (pH 7.2) and culturing was carried out at 30°C for 18 hours. The culture broth was centrifuged at 8,000 rpm for 10 minutes to cellect cells. The cells were suspended in Sample buffer of Laemmli, subjected to SDS-polyacrylamide gel electrophoresis and stained with Coomassie Brilliant Blue to detect a polypeptide band at the portion of a molecular weight of about 25,000. The band was not detected when Escherichia coli which did not carry the plasmid was used. As the result, it was confirmed that Escherichia coli carrying pSGHIB2 produced salmon growth hormone polypeptide in a large amount.

The cells were then washed twice with 30 mM Tris-HCl buffer (pH 7.5) containing 30 mM NaCl. The washed cells were suspended in 10 mℓ of the buffer described above and subjected to ultrasonic disruption using Sonifier cell disruptor 200 (Branson Sonic Power Company) under output control of 2 at 0°C for 10 minutes. The disrupted cells were centrifuged at 15,000 rpm for 30 minutes to obtain cell residues. Salmon growth hormone polypeptide was extracted and purified from the cell residues by the method of Marston et al. [F.A.O. Marston et al.: Biotechnology 2, 800 (1984)].

Reference Example 2

Production of eel growth hormone:

Escherichia coli W3110 No. 49 was transformed with recombinant plasmid pUPA1 isolated from Escherichia coli EUPA1 (FERM BP-825) by the method of Birnboim et al. [H.C. Birnboim et al.: Nucleic Acids Res. 7, 1513 (1979)]. The transformation was carried out by a conventional method [Proc. Natl. Acad. Sci., USA 69, 2110 (1972)]. The obtained ampicillin-resistant colony was inoculated in 8 mℓ of MCG medium (pH 7.2) and culturing was carried out at 30°C for 18 hours. The culture broth was centrifuged at 8,000 rpm for 10 minutes to cellect cells. The cells were suspended in Sample buffer of Laemmli, subjected to SDS-polyacrylamide gel electrophoresis and stained with Coomassie Brilliant Blue to detect a polypeptide band at the portion of a molecular weight of about 23,000. The band was not detected when Escherichia coli which did not carry the plasmid was used. As the result, it was confirmed that Escherichia coli carrying pUPA1 produced eel growth hormone polypeptide in a large amount.

The cells were then washed twice with 30 mM Tris-HCl buffer (pH 7.5) containing 30 mM NaCl. Eel growth hormone polypeptide was extracted and purified from the washed cells by the same method as in Reference Example 1.

Reference Example 3

100g of sodium casein and 10, 100, 1,000 or 10,000 mg of salmon growth hormone (SGH) were added to 400 ml of water to make a suspension.

50g of the suspension, 50g of Hokuyo gyofun - (fish meal produced by Nippon Suisan Co.), 10g of beer yeast (product of Kirin Brewery Co.), 5g of fish soluble (product of Nippon Kagaku Shiryo Co.), 5g of vitamin mixture (product of Kawai Pharmaceuticals Co.), 5g of a mineral mixture (Halver) (product of Kawai Pharmaceuticals Co.), 3g of purified cod-liver oil (Product of Nippon Kagaku Shiryo Co.), 2 g of synthetic adhesive agent CMC (product of Daicel Chemical Industries, Ltd.), and 10 g of purified soybean oil cake (product of Showa Sangyo Co.) were mixed to make 100g of a synthetic feed. As a control, a synthetic feed without growth hormone was prepared.

Reference Example 4

5g of glutelin was dissolved in 50 ml of water - (pH 2.0). 20 mg of SGH was dissolved in the solution and 50g of Hokuyo Gyofun, 15g of beer yeast, 5g of fish soluble, 5g of a vitamine mixture, 5g of a mineral mixture (Halver), 3g of purified cod-liver oil, 2g of synthetic adhesive agent and 10g of purified soybean oil cake were mixed to make 100g of a synthetic feed. As a control, a synthetic feed without SGH was prepared.

Reference Example 5

5g of zein was dissolved in 80 ml of 60% ethanol. 10 mg of eel growth hormone (eGH) was suspended in the solution and 50g of Hokuyo Gyofun, 15g of beer yeast, 5g of fish soluble, 5g of a vitamine mixture, 5g of a mineral mixture - (Halver), 3g of purified cod-liver oil, 2g of synthetic adhesive agent and 10g of purified soybean oil cake were mixed to make 100g of a synthetic feed. As a control, a synthetic feed without eGH was prepared.

Reference Example 6

Production of salmon prolactin

Natural type salmon prolactin was extracted by the method of Kawauchi et al . (General and Comparative Endocrinology 49: 446 -458, 1983) as follows.

A small amount of ice water was added to 50g of the pituitary gland of Oncorhynchus keta and the mixture was subjected to grinding with Silverson homogenizer (product of Machines Co.) under ice cooling. 150 ml of HCl-acetone (1:28 mixture, V/V) cooled at -20°C was added to the ground solution. The mixture was subjected to extraction with stirring by a magnetic stirrer at 0°C for one hour and centrifugation at 12,000 rpm for 30 minutes to separate an extract and a residue. 100 ml of 80% acetone was added to the residue and the mixture was subjected to extraction with stirring by a magnetic stirrer at 0°C for one hour and centrifugation at 12,000 rpm for 30 minutes to separate an extract and a residue.

Both extracts were combined and 3l of cooled acetone was added. The mixture was allowed to stand at -20°C for 15 hours. The precipitate collected by centrifugation at 10,000 rpm for 20 minutes was dried in a desiccator containing NaOH pellets.

1.5g of the acetone powder was dissolved in 36 ml of 0.1N acetic acid and passed through a column (5.5 x 68 cm) of Sephadex G25 (Pharmacia Fine Chemicals Inc.). The eluates were collected and freeze-dried.

0.9g of the freeze-dried product was dissolved in 100 ml of water (pH 3.0) and the pH was adjusted to 4.4 with 0.1N NaOH. The precipitate formed was removed by centrifugation at 15,000 rpm for 30 minutes. the supernatant was passed through a column (1 x 30 cm) containing Carboxymethyl-Sephadex C-25 (product of Pharmacia Fine Chemicals Inc.) equilibrated with 0.05M aqueous ammonium acetate (pH 4.6).

The column was washed with 150 ml of 0.05M aqueous ammonium acetate (pH 4.6) and elution was carried out with a gradient of 0.05 -0.2M aqueous ammonium acetate. The eluate was collected in 3 ml fractions. Salmon prolactin was contained in fractions No. 85 to 92. The fractions were combined and passed through a column (2.64 x 47 cm) containing Sephadex G 100 (product of Pharmacia Fine Chemicals Inc.) equilibrated with 0.05M aqueous ammonium acetate (pH 8.0). The eluate was freeze-dried to obtain 90 mg of a prolactin powder.

## Claims

1. A method for culturing fishes or shellfishes by administering a growth hormone of a fish or a bird to the fishes or shellfishes.

2. The method according to claim 1, wherein the growth hormone is administered by dipping the fishes or shellfishes in a solution or a suspension containing the growth hormone.

3. The method according to claim 1, wherein the growth hormone carried homogeneously on a water insoluble carrier is administered orally.

4. The method according to claim 1, wherein the growth hormone is that extracted from a pituitary gland of a salmon or an eel.

5. The method according to claim 1, wherein the growth hormone is that obtained by recombinant DNA technology.

6. A feed for culturing fishes, which contains a fish growth hormone carried homogeneously on a water insoluble carrier.

7. A feed for culturing shellfishes, which contains a fish growth hormone.